# EUROPEAN PATENT SPECIFICATION

(11) **EP 3 675 965 B1**
(45) Date of publication and mention of the grant of the patent: **17.04.2024**
(21) Application number: 18765408.2
(22) Date of filing: 30.08.2018
(51) Int. Cl.: A62B 18/02, A62B 9/00, A61M 16/00, A62B 18/00, A62B 7/10, A61M 16/06, A61M 16/10

(54) **BREATHING MASK**
ATEMMASKE
MASQUE RESPIRATOIRE

(30) Priority: 01.09.2017 WO PCT/CN2017/100182; 02.11.2017 EP 17199723
(43) Date of publication of application: 08.07.2020
(73) Proprietor: Koninklijke Philips N.V., 5656 AG Eindhoven (NL)
(72) Inventor: MCGARVA, John, Robert, 5656 AE Eindhoven (NL); WEI, Huibin, 5656 AE Eindhoven (NL); KELLY, Declan, Patrick, 5656 AE Eindhoven (NL); REEKERS, Ruben, Arnold, Herman, 5656 AE Eindhoven (NL)
(74) Representative: Philips Intellectual Property & Standards
(86) International application number: PCT/EP2018/073385
(87) International publication number: WO 2019/043118

(56) References cited:
- WO-A1-92/19322
- WO-A1-2013/067592
- CN-U- 206 499 795
- CN-Y- 2 300 426
- DE-B- 1 234 531
- GB-A- 2 264 649
- US-A1- 2006 048 778
- US-A1- 2017 080 263

## Description

### FIELD OF THE INVENTION

This invention relates to breathing masks.

### BACKGROUND OF THE INVENTION

Air pollution is a worldwide concern. The World Health Organization (WHO) estimates that 4 million people die from air pollution every year. Part of this problem is the outdoor air quality in cities. Nearly 300 smog-hit cities fail to meet national air quality standards.

Official outdoor air quality standards define particle matter concentration as mass concentration per unit volume (e.g. µg/m³). A particular concern is pollution with particles having a diameter less than 2.5 µm (termed "PM2.5") as they are able to penetrate into the gas exchange regions of the lung (alveoli), and very small particles (<100 nm) may pass through the lungs to affect other organs.

Since this problem will not improve significantly on a short time scale, a common way to deal with this problem is to wear a mask which provides cleaner air by filtration and the market for masks in China and elsewhere has seen a great surge in recent years. For example, it is estimated that by 2019, there will be 4.2 billion masks in China.

However, during use, the temperature and relative humidity inside the mask increases and combined with the pressure difference inside the mask relative to the outside, makes breathing uncomfortable. To improve comfort and effectiveness, a fan can be added to the mask which draws in air through a filter. For efficiency and longevity reasons these are normally electrically commutated brushless DC fans.

The benefit to the wearer of using a powered mask is that the lungs are relieved of the slight strain caused by inhalation against the resistance of the filters in a conventional non-powered mask.

Furthermore, in a conventional non-powered mask, inhalation also causes a slight negative pressure within the mask which leads to leakage of the contaminants into the mask, which leakage could prove dangerous if these are toxic substances. A powered mask delivers a steady stream of air to the face and may for example provide a slight positive pressure, which may be determined by the resistance of an exhale valve, to ensure that any leakage is outward rather than inward.

Fan assisted masks thus may improve the wearing comfort by reducing the temperature, humidity and breathing resistance. Fan assisted masks may be provided with an inhale fan or an exhale fan or both. An inhale fan assists in drawing air through a filter and enables a positive mask pressure to be obtained to prevent contaminants leaking into the mask volume. An exhale fan assists in the mask ventilation and ensures the breathed out carbon dioxide is fully expelled.

However, in a cold weather, this active ventilation, particularly when use is made of an inhalation fan, will directly bring in air at the ambient temperature, which is rather low.

A first issue is that the humidity level inside the mask is typically relatively high due to the breathing of the user. The exhaled humid air (100% relative humidity at 37 degrees Celsius) in a cold mask will immediately cause the condensation of the water vapor inside the mask. This condensation may be uncomfortable or unpleasant for the user of the mask.

A second issue is that when breathing in air temperatures that are low, the coldness and dryness of the air can cause the muscles around the airway to tighten as the body attempts to restrict the flow of the cold air into the lungs. This airway narrowing is sometimes referred to as exercise asthma, or cold air-induced asthma, and restricts the normal volume of air retrieved in an inhalation. If a person already has a lung disease, or a condition like asthma, the effect of the cold air on the lungs can further exacerbate breathing difficulties. This may lead to even more restricted airflow into the lungs.

WO 2013/067592 and DE1234531 each disclose a respirator with a heat exchange arrangement. US 2017/080263 discloses a breathing mask.

There is therefore a need for a powered (fan assisted) mask design which avoids the problem of drawing in cold ambient air into the mask.

### SUMMARY OF THE INVENTION

The invention is defined by the claims.

According to examples in accordance with an aspect of the invention, there is provided a breathing mask comprising:
a mask body for covering the nose and/or mouth of a user thereby defining a mask chamber;
a filter;
an air pump for ventilating the mask chamber; and
- an air heat exchanger for transferring heat from air exhaled from the mask chamber to air drawn in to the mask chamber,
wherein the air heat exchanger comprises a first set of air flow channels in series with the filter for providing filtered outside air to the mask volume/chamber, referred to as inhaled air, and a different second set of air flow channels for expelling air from the mask volume/chamber to the outside, referred to as exhaled air, and a thermal coupling between the first and second sets of air flow channels.

The use of different sets of air flow channels for providing filtered outside air to the mask volume/chamber and for expelling air from the mask volume/chamber to the outside allows for a more simple design whereby additional filter structures inside the mask volume/chamber are not needed anymore.

According to an embodiment, the first and the second sets of air flow channels have direct access to the mask chamber to allow filtered outside air to enter the mask chamber and to allow exhaled air to be expelled from the mask chamber. The mask chamber is the chamber where exhaled air from the user immediately ends up after exhalation.

This mask design provides thermal coupling between the inhaled air and the exhaled air. By providing the flows in different channels, mixing between the two air flows is prevented and more effective thermal coupling between the flow channels is made possible. Hence, the air heat exchanger is adapted or constructed such that mixing of the two air flows does not, or in a very limited amount, occur. The heating of the inhaled air reduces condensation in the mask volume and also improves comfort for the user by preventing inhalation of very cold ambient air.

Thus, the air heat exchanger is configured such that mixing of inhaled air and exhaled air is prevented. This is realized by providing a first set of air flow channels for providing filtered outside air to the mask volume and a separate second set of air flow channels for expelling air from the mask volume to the outside. The first set and the second set of air flow channels are constructed or manufactured such that air flowing through the first set of air flow channels does not, or only in a very limited amount, reach the air inside the second set of air flow channels while flowing through the first set of air flow channels. Further, the first set and the second set of air flow channels are constructed or manufactured such that air flowing through the second set of air flow channels does not, or only in a limited amount, reach the air inside the first set of air flow channels while flowing through the second air flow channels. The first and second set of air flow channels are constructed or manufactured such that a transfer of heat takes place and no significant transfer of air occurs between the air flow channels.

The heat exchanger may not only allow heat to pass from one flow channel to the other but also moisture (latent heat transfer). This depends on the materials of construction of the heat exchanger. It may be desirable to allow moisture from the exhaled air to be incorporated into the incoming air to improve the comfort of the breathed-in air. The materials should not allow significant cross membrane transport of carbon dioxide but permit transport of water vapor.

According to an embodiment, the air pump is coupled to the air heat exchanger. The presence of first and the second air flow channels in the air heat exchanger may restrict inhalation and/or exhalation of the user. To allow efficient heat exchange and reduce restriction of the inhalation and/or exhalation of the user, the air pump is coupled to the heat exchanger. The air pump ensure that enough air is imported or expelled in/from the mask chamber to increase comfort of the user and increase heat exchange efficiency.

According to an embodiment, the air pump is coupled to the first set of air flow channels.

According to an embodiment, the air pump is coupled to the second set of air flow channels.

According to an embodiment, the air pump comprises two pumps whereby a first pump is connected to the first set of air flow channels and imports air into the mask and whereby a second pump is connected to the second set of air flow channel and expels air from the mask.

The mask body is for example impermeable and has an opening for the filter and air heat exchanger. Thus, all air drawn in to the mask volume is thermally treated using heat extracted from the exhaled air.

The air pump may comprise:
an inhalation air pump; or
an exhalation air pump; or
an inhalation and an exhalation air pump.

An inhalation air pump enables a positive pressure to be maintained in the mask volume and provides assistance in breathing in through the filter. An exhalation air pump provides effective ventilation of the mask volume. When both are provided, the timing of operation of the two air pumps may be synchronized with the breathing cycle of the user. A pressure sensor may be provided for sensing the pressure inside the mask, or the differential pressure between the inside and outside, to enable the breathing cycle timing to be obtained.

According to an embodiment, an inhalation pump (for importing air into the mask chamber) is coupled to the first set of air flow channels. It is an advantage of the invention that the air heat exchanger is constructed such that mixing of air between the first and second air flow channels is prevented as this allows an efficient operation of the inhalation pump leading to lower consumption. Due to this construction of the air heat exchanger it can be ensured that only air inside the first set of air flow channels is propagated through the first set of air flow channels and supplied to the user without or with little pressure loss or contamination with air from the second set of air flow channels.

According to an embodiment, an exhalation pump (for expelling air from the mask chamber) is coupled to the second set of air flow channels. It is an advantage of the invention that the air heat exchanger is constructed such that mixing of air between the first and second air flow channels is prevented as this allows a more efficient operation of the exhalation pump leading to lower power consumption. Due to this construction of the air heat exchanger it can be ensured that air inside the second set of air flow channels is propagated through the second set of air flow channels and expelled from the mask without or with little pressure loss.

According to an embodiment, an inhalation pump (for importing air into the mask chamber) is coupled to the first set of air flow channels and an exhalation pump (for expelling air from the mask chamber) is coupled to the second set of air flow channels. It is an advantage of the invention that the air heat exchanger is constructed such that mixing air between the first and second air flow channels is prevented as this allows an efficient operation of the inhalation and exhalation pump, leading to lower power consumption of both pumps. Due to this construction of the air heat exchanger it can be ensured that air inside the first set of air flow channels is propagated through the first set of air flow channels and supplied to the user without or with little pressure loss or contamination with air from the second set of air flow channels. Also, due to this construction of the air heat exchanger it can be ensured that air inside the second set of air flow channels is propagated through the second set of air flow channels and expelled from the mask without or with little pressure loss. Further, due to the construction of the air heat exchanger, the operation of the inhalation pump is not or little affected by the operation of the exhalation pump. This may be advantageous in a situation where both pumps remain active at lower power during certain phases of the breathing cycle.

According to an embodiment, the first and the second set of air flow channels are comprised in a stacked arrangement. The first set of air flow channels may be stacked onto the second set of air flow channels or vice versa. There may be multiple layers present of the first and the second set of air flow channels.

According to an embodiment, the direction of the first set of air flow channels is different than the direction of the second set of air flow channels. This allows for a spaced placement of inlets and outlets of the mask which are connected to the first or second air flow channels.

In one arrangement, the air flow channels are all parallel to each other, forming an array of channels. Some channels may be grouped to provide inhalation flow and others may be grouped to provide exhalation flow.

In another arrangement, the first set of air flow channels is orthogonal to the second set of air flow channels. This makes the flow arrangement easier to form, because the flow channels terminate at different spatial locations. Thus, the filter may be provided only at the inlet side, and the connections to the air pump arrangement are more easily made since all channels at any location may be connected together.

For example, the air heat exchanger may comprise:
a first external face and a parallel first internal face, with the first set of flow channels between the first external and internal faces; and
a second external face and a parallel second internal face, with the second set of flow channels between the second external and internal faces, the first faces perpendicular to the second faces.

The filter may then be provided at the first external face.

The air pump may comprise:
an air pump at the first external face; or
an air pump at the second external face; or
a first air pump at the first external face and a second air pump at the second external face.

The air pump, or both air pumps, may for example comprise a fan or fans.

The air heat exchanger for example comprises a mesh of thermally conductive walls between which the air flow channels are defined. The walls are for example made of modified paper, or gel, or very thin metal layers. Modified metal layers may be used with polymer coatings, or graphites may be used.

At least some of the flow channels may comprise a desiccant. This is used to extract moisture from the exhaled air and thereby generate additional heat for the thermal coupling.

Instead of using a fan or fans, the air pump may comprise a micropump comprising an array of pump channels. This provides a more compact air pump than a fan arrangement.

A controller may then be provided which is adapted to control the pump channels to provide a pump channel flow in a selectable direction, wherein the controller is adapted to configure first pump channels with a first flow direction to form the first set of air flow channels and configure second pump channels with a second flow direction to form the second set of air flow channels.

In this way, the first and second pump channels are dynamically controllable. A single micropump design may then function both as an inhalation pump and an exhalation pump.

In one configuration, the controller arranges the first pump channels to surround the second pump channels. In this way, maximum heat transfer is obtained from the second pump channels to the surrounded first pump channels.

The air heat exchanger for example comprises a stack of corrugated layers. Alternate layers may be orthogonal to each other. This provides a simple low cost air heat exchanger design.

At least some of the pump channels may comprise a desiccant (as mentioned above), and the controller may then be adapted to control the flow direction to implement regeneration of the desiccant. The micropump and the air heat exchanger may comprise a shared array of channels thus forming a single unit.

These and other aspects of the invention will be apparent from and elucidated with reference to the embodiment(s) described hereinafter.

### BRIEF DESCRIPTION OF THE DRAWINGS

Examples of the invention will now be described in detail with reference to the accompanying drawings, in which:
Fig. 1 shows in schematic form a mask in accordance with one example of the invention;
Fig. 2 plots the humidity ratio relative to dry air versus temperature inside a mask when the outdoor air is at 10 degrees Celsius and 60% relative humidity;
Fig. 3 plots the humidity ratio relative to dry air versus temperature inside a mask when increased ventilation is used;
Fig. 4 plots the humidity ratio relative to dry air versus temperature inside a mask when using the heat exchange function;
Fig. 5 shows one example of the mask design in more detail;
Fig. 6 shows one possible design for the air heat exchanger;
Fig. 7 shows another possible design for the heat exchanger;
Fig. 8 shows an alternative air pump arrangement in the form of a honeycomb array of pump channels; and
Fig. 9 shows an arrangement of parallel channels which may form the micropump arrangement as well as the heat exchanger.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a breathing mask having an air heat exchanger for transferring heat from air exhaled from a mask chamber to air drawn in to the mask chamber. The air heat exchanger comprises a first set of air flow channels in series with the filter for providing filtered outside air to the mask volume and a different second set of air flow channels for expelling air from the mask volume to the outside, and a thermal coupling between the first and second sets of air flow channels.

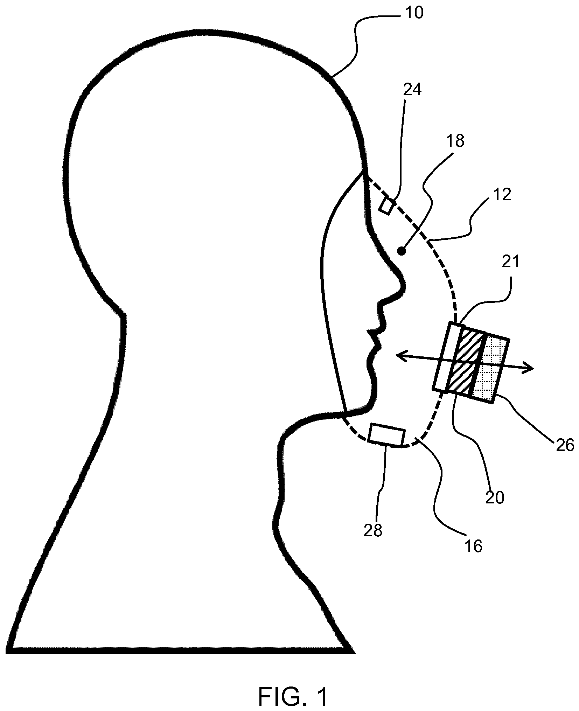

Fig. 1 shows a subject 10 wearing a face mask 12 which covers the nose and mouth of the subject. The purpose of the mask is to filter air before it is breathed in by the subject and to provide active control of the flow of air into an air chamber 18 (i.e. the mask volume) and/or out of the air chamber. An air pump arrangement 20 provides active flow control. The air pump arrangement may be a fan arrangement (of one or multiple fans) or it may be an arrangement of pump channels forming a micropump, as explained below.

A filter 21 is provided in series with the air pump arrangement 20. The body 16 of the mask 12 is preferably impermeable (to air) so that all outside air is drawn through the filter. The mask has a controller 28 for controlling the air pump arrangement.

The example shown has bidirectional air pumping.

Air is drawn in to the air chamber 18 by inhalation, and with assistance provided by the air pump arrangement.

During exhalation, air is expelled from the air chamber 18. In this schematic example, exhalation is through the same air pump arrangement and hence also through the filter 21 (even though the expelled air does not need to be filtered).

The operation of the air pump arrangement is preferably also timed with the breathing cycle of the user. For this purpose, a pressure sensor 24 is provided either for measuring the pressure inside the air chamber 18 or for measuring a differential pressure between the inside and outside of the air chamber.

Various mask designs are possible, with assisted inhalation and/or assisted exhalation, and with different filter designs. When there is both assisted inhalation and exhalation, there may be a single air pump arrangement or separate inhale and exhale air pumps.

When the user breathes out, the exhaled air flow typically has a body temperature of 37 degrees Celsius and 100% relative humidity. This results in condensation when cold ambient air is drawn in.

The invention provides an air heat exchanger 26 for transferring heat from air exhaled from the mask chamber to air drawn in to the mask chamber. As explained further below, the air heat exchanger comprises a first set of air flow channels in series with the filter 21 for providing filtered outside air to the mask volume and a different second set of air flow channels for expelling air from the mask volume to the outside. The expelled air may also pass through the filter or the second set of air flow paths may not pass the filter. The two sets of flow paths are thermally coupled. By providing the flows in different channels, mixing between the two air flows is prevented and more effective thermal coupling between the flow channels is made possible. The heating of the inhaled air reduces condensation in the mask volume and also improves comfort for the user by preventing inhalation of very cold ambient air.

The filter and heat exchanger are provided in an opening of the body of the mask. As mentioned above, the air pump may comprise an inhalation air pump or an exhalation air pump or both.

During winter, or even spring and autumn, it is impossible to prevent condensation in regular masks as the warm and humid exhaled air mixes with cold incoming air. The outgoing air is full of water vapor which then condenses within the mask volume 18.

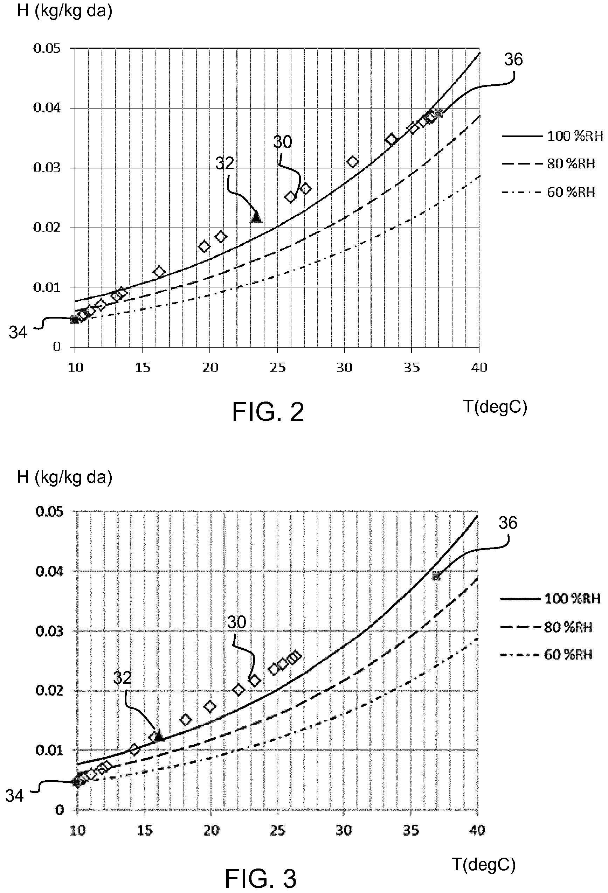

Fig. 2 plots the humidity ratio relative to dry air (kg/kg da) versus temperature when the outdoor air is at 10 degrees Celsius and 60% relative humidity (RH) which are typical temperate conditions in spring and autumn. The three graph lines show the humidity of air as a function of temperature for three relative humidity values (60%, 80% and 100%).

Plot 30 shows the mask internal condition at different times during single inhalation and exhalation breathing cycle, point 32 shows a weighted average, with weighting based on the amount of air which passes through the mask cavity for each point in the breathing cycle. Thus, it represents an overall condition in the mask volume. Point 34 shows the outdoor conditions, and point 36 shows the human breath condition.

Fig. 2 shows that the weighted average condition of point 32 in the mask is above 100% saturation (i.e. the solid graph line) leading to condensation.

It is known to seek to solve this problem by providing additional ventilation into the mask using a fan system either to blow air into the mask or to draw it out. However there are problems with this ventilation, in that the cold ventilated air is unpleasant to the user (so only applicable in summer) and it may still not be sufficient to prevent condensation.

Fig. 3 shows that ventilation cannot completely mitigate the condensation issue. As for Fig. 2, plot 30 shows the mask internal conditions during a breathing cycle, point 32 shows the weighted average, point 34 shows the outdoor conditions, and point 36 shows the human breath condition.

The weighted average condition of the air inside the mask is biased towards the outdoor air condition. However even with this level of fan ventilation (set at 3 times the user's breathing volume) the weighted average condition of the air inside the mask is still above the 100% RH saturation point. The problem is even worse if the outdoor conditions are colder, for example in winter.

This condensation is unpleasant to the user and prevents the user from wearing the mask for an extended time.

The air heat exchange unit overcomes this disadvantage by pre-heating the incoming air, before it enters the mask. This is achieved by extracting heat from the outgoing air.

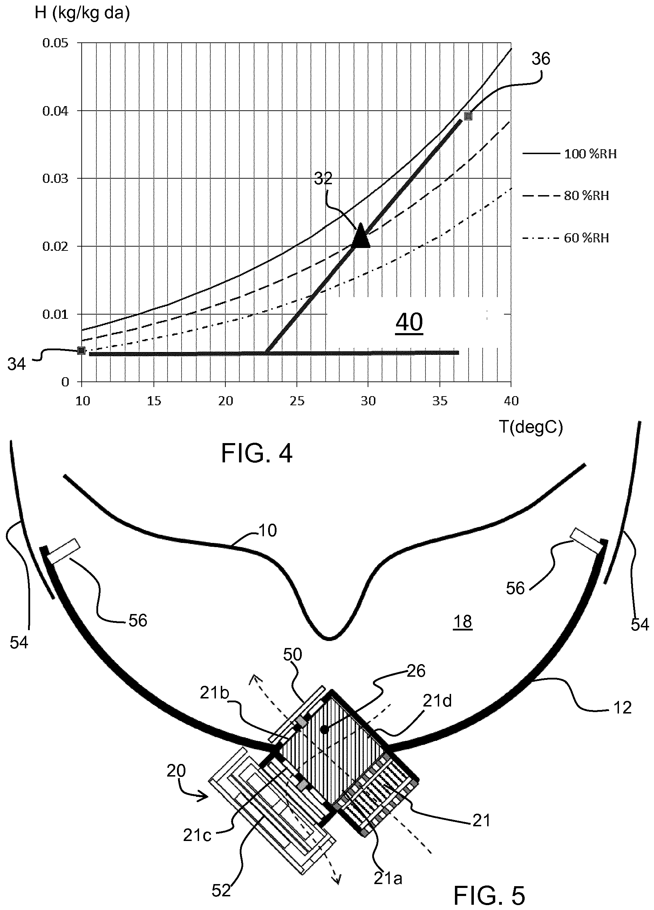

Fig. 4 shows the influence of performing a heat exchange function. Firstly, the hot breathed out air will condense moisture into the cold heat exchanger. When the heat exchanger 26 is outside the mask, the moisture can be disposed of safely. Secondly, the heat exchanger 26 is now warm and air breathed in will be heated by sensible heat transfer (i.e. with no change in phase), thus reducing its relative humidity and its ability for the hot humid air remaining in the mask volume to condense. There may also be latent heat transfer (i.e. associated with phase change) depending on the design of the heat exchanger.

Point 34 again shows the outdoor conditions, and point 36 shows the human breath condition. The region 40 is where there is heating of incoming air in the heat exchanger.

Fig. 4 shows that the outside air is heated up to a maximum of 37 degrees Celsius by the heat exchanger. However as the heat exchanger cools down, the final air entering the mask will be at the outdoor condition. Thus the average temperature of the incoming air (point 32) will be mid-way between 37 degrees Celsius and the ambient outdoor temperature. When this air mixes with the exhaled breath, the weighted average condition in the interior of the mask is at a much lower relative humidity that can prevent condensation.

Fig. 5 shows one example of the mask design in more detail.

It shows the air heat exchanger 26 in more detail. It comprises a 3 dimensional slab, for example a cuboid. It has a first external face 21a and a parallel first internal face 21b, with the first set of flow channels between the first external and internal faces 21a, 21b. There is an inhale check valve 50 at the inside face.

There is a second external face 21c and a parallel second internal face 21d, with the second set of flow channels between the second external and internal faces 21c, 21d. There is an exhale check valve 52 at the inside face.

The first faces 21a, 21b are perpendicular to the second faces 21c, 21d. This orthogonal arrangement realizes a maximum contact for heat exchange.

The air pump 20 in this design is an exhale fan with a local battery, fan motor, fan blade and control PCB.

Fig. 5 also shows a strap 54 for holding the mask and a mask seal 56.

The design of Fig. 5 avoids a direct air flow into the face. The inlet air will always be directed from a side or from above or from below to the nose, which also improves the wearing comfort.

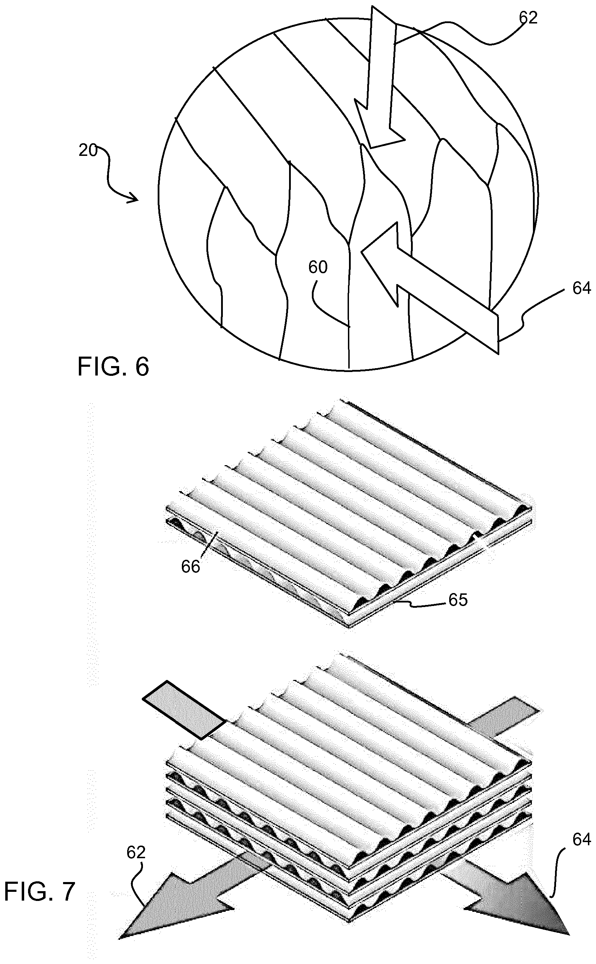

Fig. 6 shows one possible design for the air heat exchanger. It comprises a mesh of thermally conductive walls 60 between which the air flow channels are defined. As shown, the mesh forms orthogonal isolated channels so that two orthogonal flow paths 62, 64 are formed.

Fig. 7 shows an alternative heat exchanger design which is formed as a stack of corrugated sheets 65, 66 oriented at 90 degrees, and stacked to form the orthogonal heat exchanging passages 62, 64. The top image shows the two types of corrugated layer, and the bottom image shows that a stack may comprise many such layers. There is sensible heat transfer through the walls of the layers. By allowing H2O latent heat penetration (but blocking the passage of CO2), there can also be H2O latent heat transfer.

The structure material to form the multiple layers of the heat exchange component is preferably very thin and has a high heat capacity, which can transfer the heat from the exhalation channels to the inhalation channels. It may also provide water absorption to reduce the possible condensation, or it may also have a porous structure to contain the condensed water. For instance, modified paper or gel may be used which could transfer the humidity as well as the heat, from the exhaled air to air for inhalation. Thin metal layers, for example modified with polymer coatings may be used. Thin materials may be used (e.g. tens or hundreds of µm), and the whole structure can even be flexible or foldable, and may for example be fixed on a frame on the mask.

In these designs, the first set of air flow channels is orthogonal to the second set of air flow channels. This makes the flow arrangement easier to form, because the flow channels terminate at different spatial locations. It also provides improved thermal coupling. The arrangement shown in Fig. 5 may be formed with this design of air heat exchanger in combination with a fan or fans. The heat exchanger thus comprises thin layers forming a set of channels.

Instead of orthogonal channels, there may be parallel channels. For example, the odd channels may be connected to one air pump (which may again be a fan), and the even channels may be connected to another air pump (which may again be a fan). Alternatively, there may be only one fan. The inlet and outlet air flow may then be in opposite directions. The heat will transfer from the exhaled air to the air for inhalation in the neighboring channel, without direct contact and contamination.

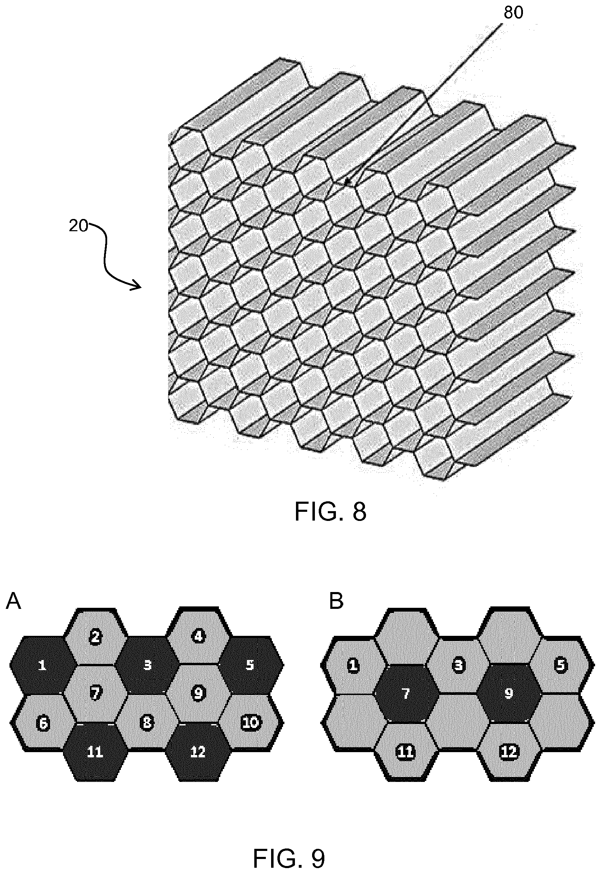

Fig. 8 shows an alternative air pump arrangement 20 the form of a honeycomb array of pump channels 80. This provides a compact micropump arrangement. The pump channels 80 may be independently controllable or controlled in groups. The channels 80 are shown as parallel and they may again be grouped into rows and columns.

Each pump channel 80 may have a controllable pump flow direction. Thus, the single pump channel arrangement functions both as an inhale (inlet) and exhale (exhaust) pump. There may be only pump channels controlled to provide outward flow during exhalation (as determined by the controller 28) and only pump channels controlled to provide inward flow during inhalation, but there could also be inward and outward flow at the same time at different flow channels.

As shown schematically in Fig. 8, every cell of the honeycomb comprises an individual pump cell and they may be controlled separately or connected for a simple implementation. One simple example is that the control terminals for the cells are connected together in each row, and different rows are separately controlled. For example, rows 1, 2, 3 and 4 could be controlled at the same time to make these rows act as intake units, whereas rows 5, 6, 7, and 8 could be controlled at the same time as discharge units. Each row will have several lines connected (for example 3) to the controller 28.

Typically, one terminal is connected to a positive voltage, another terminal is connected to a negative voltage, and a third terminal is connected to a control voltage that could be positive or negative.

By switching the third terminal, a diaphragm film of each single cell moves towards the first or second terminal by electrostatic attraction and repulsion. This generates the desired micro flow. By controlling the sequence of signals applied to the third terminal, the flow rate is controlled, and by switching the first and second terminals the direction of flow also can be controlled.

For a full implementation there could be more than three terminals for each cell for better performance. There are insulation layers between each channel.

By way of example, each cell is hexagonal with the first and second terminals on opposite faces, and the third terminal arranged to move between them.

The pump channels are all parallel in this example, but there could instead be pump channels with different directions. The micropump and the heat exchanger may be formed as a combined unit, with the channels being used for micropump operation as well as for heat exchange.

At least some of the flow channels may comprise a desiccant, in particular flow channels which are at least at some points in time used for the exhale flow. This is used to extract moisture from the exhaled air and thereby generate additional heat for the thermal coupling. Other functional layers may also be used further purify the air. In particular, in very cold weather condensation will always happen because the heat transferred from the exhaled air is not enough to warm up the air for inhalation. The desiccant absorbs humidity from the exhaled air and generates heat, which could better warm the air for inhaling, without any extra energy input and further reduce the condensation from the exhaled air.

The air for inhalation may be moist if the ambient air is very dry. If there is condensation from the exhaled air, and the structural material between layers absorbs water, the pumped-in ambient air will increase in humidity, which could also reduce the irritation from dry air.

When a micropump design is used, the heat exchange component may be made smaller with higher heat exchange efficiency. A single micropump channel has a very small dimension (e.g. 50 µm), and an array of several hundreds or thousands of single micropump channels may be used to provide enough air flow for human breath. The single micropump channels may be formed into any pattern and can could alternate between two directions of air flow by switching the pumping directions.

The heat exchange component can be structured in the same array structure or integrated together with the micropump array (or the micropump channels could be extended with the heat exchange material). Because of the small dimension of the channels, the contact area between the warmer air and cooler air is greatly enlarged, compared to a structure of parallel layers.

Fig. 9 shows an arrangement of parallel channels which may form the micropump arrangement as well as the heat exchanger. The lightly shaded channels are configured for exhalation flow (i.e. from warm to cold) and the darkly shaded channels are configured for inhalation flow (from cold to warm). The flow in the two channel sets are in opposite directions. In Fig. 9A, the channels containing the warmer air are surrounded by the cold channels, so that the heat is transferred with a higher efficiency from one warm channel to a set of surrounding colder channels.

Due to the bi-directional function of the micropump, another option could be adopted under certain conditions, in particular by reversing the pumping direction of selected channels to achieve a better performance.

For example, when a desiccant or other functional layer is used to absorb condensation and generate heat, this layer may need regeneration when saturated. For example, if the darkly shaded channels in Fig. 9A reach adsorption saturation, the pumping directions could be changed to the configuration shown in Fig. 9B where the exhaled air channels pass through two channels previously forming inhalation channels. The channels to be regenerated are used for drawing air for inhalation. In this way, the chemicals in channels 7 and 9 can continue to have a moisture reducing function, and the chemicals in channels 1, 3, 5, 11, 12 can be regenerated by the cooler and drier air.

In this way, the controller of the mask may control the pump channels to provide a pump channel flow in a selectable direction, with dynamically controllable first and second pump channels. The controller controls the flow direction to implement the required regeneration of the desiccant.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. An air pollution filtering breathing mask for filtering air before it is breathed in by a subject, comprising:
a mask body (12) for covering the nose and/or mouth of a user (10) thereby defining a mask chamber (18);
a filter (21);
an air pump (20) for ventilating the mask chamber (18); and
an air heat exchanger (26) for transferring heat from air exhaled from the mask chamber (18) to air drawn in to the mask chamber (18),
wherein the air heat exchanger (26) comprises a first set of air flow channels in series with the filter (21) for providing filtered outside air to the mask volume (18) and a different second set of air flow channels for expelling air from the mask volume (18) to the outside and whereby mixing between the two air flows is prevented, and a thermal coupling between the first and second sets of air flow channels, wherein the air heat exchanger (26) comprises a mesh of thermally conductive walls between which the air flow channels are defined.

2. A breathing mask as claimed in claim 1, wherein the mask body (12) is impermeable and has an opening for the filter (21) and air heat exchanger (26).

3. A breathing mask as claimed in claim 1 or 2, wherein the air pump (20) comprises:
an inhalation air pump; or
an exhalation air pump; or
an inhalation and an exhalation air pump.

4. A breathing mask as claimed in any preceding claim, wherein the channels of the first set of air flow channels are orthogonal to the channels of the second set of air flow channels.

5. A breathing mask as claimed in claim 4, wherein the air heat exchanger (26) comprises:
a first external face and a parallel first internal face, with the first set of flow channels between the first external and internal faces; and
a second external face and a parallel second internal face, with the second set of flow channels between the second external and internal faces, the first faces perpendicular to the second faces.

6. A breathing mask as claimed in claim 5, wherein the filter (21) is provided at the first external face.

7. A breathing mask as claimed in claim 5 or 6, wherein the air pump (20) comprises:
an air pump (20) at the first external face; or
an air pump (20) at the second external face; or
a first air pump at the first external face and a second air pump at the second external face.

8. A breathing mask as claimed in any one of claims 1 to 7, wherein the air heat exchanger (26) comprises a stack of corrugated layers.

9. A breathing mask according to any of the preceding claims, wherein the air pump (20) is coupled to the air heat exchanger (26).

10. A breathing mask as claimed in any preceding claim, wherein the air pump (20) comprises a micropump comprising an array of pump channels.

11. A breathing mask as claimed in claim 10, wherein each pump channel has a controllable pump direction, wherein the breathing mask further comprises a controller which is adapted to control the pump channels to provide a pump channel flow in a selectable direction, wherein the controller is adapted to configure first pump channels with a first flow direction to form the first set of air flow channels and configure second pump channels with a second flow direction to form the second set of air flow channels.

12. A breathing mask as claimed in claim 11, wherein the controller is adapted to configure the first pump channels to surround the second pump channels.

13. A breathing mask as claimed in any one of claims 10 to 12, wherein at least some of the pump channels comprise a desiccant, and wherein the controller is adapted to control the flow direction to implement regeneration of the desiccant.

14. A breathing mask as claimed in any one of claims 10 to 13, wherein the micropump and the air heat exchanger (26) comprise a shared array of channels.

## Patentansprüche

1. Luftverschmutzung filternde Atemmaske zum Filtern der Luft, bevor sie von einer Person eingeatmet wird, bestehend aus: einen Maskenkörper (12) zum Abdecken der Nase und/oder des Mundes eines Benutzers (10), wodurch eine Maskenkammer (18) definiert wird; ein Filter (21); eine Luftpumpe (20) zum Belüften der Maskenkammer (18); und einen Luftwärmetauscher (26) zum Übertragen von Wärme von der aus der Maskenkammer (18) ausgeatmeten Luft auf die in die Maskenkammer (18) angesaugte Luft, wobei der Luftwärmetauscher (26) einen ersten Satz Luftströmungskanäle in Reihe mit dem Filter (21) zur Bereitstellung gefilterter Außenluft für das Maskenvolumen (18) und einen anderen zweiten Satz Luftströmungskanäle zum Ausstoßen von Luft aus diesem umfasst Maskenvolumen (18) nach außen und wodurch eine Vermischung der beiden Luftströme verhindert wird, und eine thermische Kopplung zwischen dem ersten und dem zweiten Satz von Luftströmungskanälen, wobei der Luftwärmetauscher (26) ein Netz aus wärmeleitenden Wänden dazwischen umfasst in dem die Luftströmungskanäle definiert sind.

2. Atemmaske nach Anspruch 1, wobei der Maskenkörper (12) undurchlässig ist und eine Öffnung für den Filter (21) und den Luftwärmetauscher (26) aufweist.

3. Atemmaske nach Anspruch 1 oder 2, wobei die Luftpumpe (20) umfasst: eine Inhalationsluftpumpe; oder eine Ausatemluftpumpe; oder eine Einatem- und eine Ausatemluftpumpe.

4. Atemmaske nach einem der vorhergehenden Ansprüche, wobei die Kanäle des ersten Satzes von Luftströmungskanälen orthogonal zu den Kanälen des zweiten Satzes von Luftströmungskanälen sind.

5. Atemmaske nach Anspruch 4, wobei der Luftwärmetauscher (26) umfasst: eine erste Außenfläche und eine parallele erste Innenfläche, mit dem ersten Satz von Strömungskanälen zwischen der ersten Außen- und Innenfläche; und eine zweite Außenfläche und eine parallele zweite Innenfläche, wobei der zweite Satz von Strömungskanälen zwischen der zweiten Außen und Innenfläche liegt und die ersten Flächen senkrecht zu den zweiten Flächen stehen.

6. Atemmaske nach Anspruch 5, wobei der Filter (21) an der ersten Außenfläche vorgesehen ist.

7. Atemmaske nach Anspruch 5 oder 6, wobei die Luftpumpe (20) umfasst: eine Luftpumpe (20) an der ersten Außenfläche; oder eine Luftpumpe (20) an der zweiten Außenfläche; oder eine erste Luftpumpe an der ersten Außenfläche und eine zweite Luftpumpe an der zweiten Außenfläche.

8. Atemmaske nach einem der Ansprüche 1 bis 7, wobei der Luftwärmetauscher (26) einen Stapel gewellter Schichten umfasst.

9. Atemmaske nach einem der vorhergehenden Ansprüche, wobei die Luftpumpe (20) mit dem Luftwärmetauscher (26) gekoppelt ist.

10. Atemmaske nach einem der vorhergehenden Ansprüche, wobei die Luftpumpe (20) eine Mikropumpe mit einer Anordnung von Pumpenkanälen umfasst.

11. Atemmaske nach Anspruch 10, wobei jeder Pumpkanal eine steuerbare Pumprichtung aufweist, wobei die Atemmaske außerdem eine Steuerung umfasst, die dazu ausgelegt ist, die Pumpkanäle zu steuern, um einen Pumpkanalfluss in einer wählbaren Richtung bereitzustellen, wobei die Die Steuerung ist so ausgelegt, dass sie erste Pumpenkanäle mit einer ersten Strömungsrichtung konfiguriert, um den ersten Satz von Luftströmungskanälen zu bilden, und zweite Pumpenkanäle mit einer zweiten Strömungsrichtung konfiguriert, um den zweiten Satz von Luftströmungskanälen zu bilden.

12. Atemmaske nach Anspruch 11, wobei die Steuerung dazu ausgelegt ist, die ersten Pumpkanäle so zu konfigurieren, dass sie die zweiten Pumpkanäle umgeben.

13. Atemmaske nach einem der Ansprüche 10 bis 12, wobei mindestens einige der Pumpenkanäle ein Trockenmittel umfassen und wobei die Steuerung dazu ausgelegt ist, die Strömungsrichtung zu steuern, um eine Regeneration des Trockenmittels durchzuführen.

14. Atemmaske nach einem der Ansprüche 10 bis 13, wobei die Mikropumpe und der Luftwärmetauscher (26) eine gemeinsame Kanalanordnung umfassen.

## Revendications

1. Masque respiratoire filtrant la pollution de l'air pour filtrer l'air avant qu'il ne soit respiré par un sujet, comprenant: un corps de masque (12) destiné à couvrir le nez et/ou la bouche d'un utilisateur (10), définissant ainsi une chambre de masque (18); un filtre (21); une pompe à air (20) pour ventiler la chambre de masque (18); et un échangeur de chaleur (26) pour transférer la chaleur de l'air expiré de la chambre de masque (18) à l'air aspiré dans la chambre de masque (18), dans lequel l'échangeur thermique (26) comprend un premier ensemble de canaux d'écoulement d'air en série avec le filtre (21) pour fournir de l'air extérieur filtré au volume du masque (18) et un second ensemble différent de canaux d'écoulement d'air pour expulser l'air du volume du masque (18) vers l'extérieur et empêcher le mélange entre les deux flux d'air, et un couplage thermique entre le premier et le second ensemble de canaux d'écoulement d'air, dans lequel l'échangeur thermique (26) comprend un réseau de parois thermoconductrices entre lesquelles les canaux d'écoulement d'air sont définis.

2. Masque respiratoire selon la revendication 1, dans lequel le corps du masque (12) est imperméable et comporte une ouverture pour le filtre (21) et l'échangeur thermique (26).

3. Masque respiratoire selon la revendication 1 ou 2, dans lequel la pompe à air (20) comprend: une pompe à air d'inhalation; ou une pompe à air d'expiration; ou une pompe à air d'inspiration et une pompe à air d'expiration.

4. Masque respiratoire selon l'une des revendications précédentes, dans lequel les canaux du premier ensemble de canaux d'écoulement d'air sont orthogonaux aux canaux du second ensemble de canaux d'écoulement d'air.

5. Masque respiratoire selon la revendication 4, dans lequel l'échangeur thermique (26) comprend: une première face externe et une première face interne parallèle, avec le premier ensemble de canaux d'écoulement entre les premières faces externe et interne; et une deuxième face externe et une deuxième face interne parallèle, avec le second ensemble de canaux d'écoulement entre les deuxièmes faces externe et interne, les premières faces étant perpendiculaires aux deuxièmes faces.

6. Masque respiratoire selon la revendication 5, dans lequel le filtre (21) est placé sur la première face externe.

7. Masque respiratoire selon la revendication 5 ou 6, dans lequel la pompe à air (20) comprend: une pompe à air (20) sur la première face externe; ou une pompe à air (20) sur la deuxième face externe; ou une première pompe à air sur la première face externe et une deuxième pompe à air sur la deuxième face externe.

8. Masque respiratoire selon l'une des revendications 1 à 7, dans lequel l'échangeur thermique (26) comprend un empilement de couches ondulées.

9. Masque respiratoire selon l'une des revendications précédentes, dans lequel la pompe à air (20) est couplée à l'échangeur thermique (26).

10. Masque respiratoire selon l'une des revendications précédentes, dans lequel la pompe à air (20) comprend une micropompe comportant un réseau de canaux de pompage.

11. Masque respiratoire selon la revendication 10, dans lequel chaque canal de pompage a une direction de pompage contrôlable, dans lequel le masque respiratoire comprend en outre un contrôleur qui est adapté pour contrôler les canaux de pompage afin de fournir un flux de canal de pompage dans une direction sélectionnable, dans lequel le contrôleur est adapté pour configurer les premiers canaux de pompage avec une première direction de flux pour former le premier ensemble de canaux de flux d'air et configurer les seconds canaux de pompage avec une deuxième direction de flux pour former le second ensemble de canaux de flux d'air.

12. Masque respiratoire selon la revendication 11, dans lequel le contrôleur est adapté pour configurer les premiers canaux de pompage afin d'entourer les seconds canaux de pompage.

13. Masque respiratoire selon l'une des revendications 10 à 12, dans lequel au moins certains des canaux de la pompe comprennent un déshydratant, et dans lequel le contrôleur est adapté pour contrôler la direction du flux afin de mettre en œuvre la régénération du déshydratant.

14. Masque respiratoire selon l'une des revendications 10 à 13, dans lequel la micropompe et l'échangeur thermique (26) comportent un ensemble de canaux communs.
